(19) Europäisches Patentamt / European Patent Office / Office européen des brevets

(11) **EP 3 750 872 B1**

(12) **EUROPEAN PATENT SPECIFICATION**

(45) Date of publication and mention
of the grant of the patent:
**07.06.2023 Bulletin 2023/23**

(21) Application number: **19180266.9**

(22) Date of filing: **14.06.2019**

(51) International Patent Classification (IPC):
**C07C 277/06** *(2006.01)*    **C07C 279/02** *(2006.01)*
**C07D 251/60** *(2006.01)*    **C07D 251/62** *(2006.01)*

(52) Cooperative Patent Classification (CPC):
(C-Sets available)
**C07D 251/60; C07C 277/06; C07D 251/62**   (Cont.)

(54) **INCREASE OF GUANIDINE CARBONATE YIELD BY REDUCTION OF MOTHER LIQUOR DISPOSAL AND QUALITY IMPROVEMENT DUE TO INCREASE OF TEMPERATURE SPREAD**

ERHÖHUNG DER GUANIDINCARBONATAUSBEUTE DURCH REDUZIERUNG DER MUTTERLAUGENENTSORGUNG UND QUALITÄTSVERBESSERUNG DURCH ERHÖHUNG DER TEMPERATURSPREIZUNG

AUGMENTATION DU RENDEMENT EN CARBONATE DE GUANIDINE PAR LA RÉDUCTION DE L'ÉLIMINATION DE LIQUEUR MÈRE ET L'AMÉLIORATION DE LA QUALITÉ DUE À L'AUGMENTATION DE L'ÉTALEMENT DE TEMPÉRATURE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.12.2020 Bulletin 2020/51**

(73) Proprietor: **Borealis Agrolinz Melamine GmbH**
**4021 Linz (AT)**

(72) Inventors:
• **Gabriel, Herbert**
 **4021 Linz (AT)**
• **Dicke, René**
 **4021 Linz (AT)**

(74) Representative: **Maikowski & Ninnemann**
**Patentanwälte Partnerschaft mbB**
**Postfach 15 09 20**
**10671 Berlin (DE)**

(56) References cited:
**EP-A1- 3 626 706**    **AT-B- 317 918**
**US-A- 3 952 057**    **US-A- 3 984 471**

EP 3 750 872 B1

(52) Cooperative Patent Classification (CPC): (Cont.)

C-Sets
**C07C 277/06, C07C 279/02**

**Description**

**Description**

The present invention relates to a method for increasing the guanidine carbonate yield in a guanidine carbonate treatment process, in particular in a combined melamine process and guanidine carbonate treatment process.

Guanidine carbonate (GC) is increasing in demand due to a broadening range of applications, especially in the cosmetic industry. Worldwide, there are not many producers of guanidine carbonate and therefore it is strategically seen as a very important product.

There are various ways of guanidine carbonate production. According to DE938843C or US2221478A guanidine salts can be obtained from dicyandiamide and ammonia salts. According to DE1593472 A1 guanidine carbonate can be synthesized from cyanamide with ammonium carbonate. Here, ammonium carbonate is mixed with aqueous ammonia and heated to a temperature between 130 and 140 °C. Then the cyanamide is added within approx. 6 minutes in a ratio of cyanamide : ammonium carbonate = 1 : 1,9. After 15 minutes the reaction mixture is cooled down to room temperature. If 120 °C is used as reaction temperature then the reaction time must be increased to 40 minutes. The yield of this synthesis is 90 %.

Guanidine carbonate may also be obtained as a by-product of the melamine low-pressure process as a white powder. Here, melamine mother liquor from a melamine low pressure process is transferred to a melamine precipitation stage wherein melamine (and melamine-by-products like ammeline, ammelide) is precipitated. The remaining liquor is transported to a guanidine carbonate precipitation stage wherein guanidine carbonate is precipitated. This is described for example in AT317918B and AT336035B both referring to methods for obtaining guanidine carbonate in the melamine low pressure process. AT336035B mentions also that the remaining guanidine carbonate mother liquor can be recycled to the evaporation stage. US 3 952 057 refers to a process for obtaining guanidine carbonate from aqueous mother liquors from a melamine producing process, wherein the aqueous solution is heated to expel ammonia and carbon dioxide, evaporating the resulting solution until the solution (after cooling to 20-30°C) is saturated with urea, separating the resulting precipitate from the solution, suspending the remaining solution in an amount of ammonia and separating undissolved guanidine carbonate.

Currently, guanidine carbonate is precipitated from melamine mother liquor by adding gaseous ammonia. After separation of the precipitated guanidine carbonate, approx. 40 % of the remaining mother liquor is disposed (part of -1200 l/h of waste water), the rest is recycled to the guanidine carbonate process.

The reason for the disposal of the guanidine carbonate mother liquor (GCML) is to meet the guanidine carbonate product specifications. However, analysis showed that only about 60% of the dissolved guanidine carbonate in the mother liquor is precipitating in guanidine carbonate precipitation stage, i.e. a large amount of guanidine carbonate remains dissolved. A mass balance of the current situation in the plant shows that indeed more than 40 % of the formed guanidine carbonate is lost by disposal of mother liquor.

Besides guanidine carbonate there are also large amounts of melamine, urea and melamine by-products (e.g. ammeline, ammelide, cyanuric acid, cyano melamine) dissolved in the mother liquor which contribute to the waste water problem.

An important criteria in the guanidine carbonate treatment process is to keep the precipitation temperature of melamine below the precipitation temperature of guanidine carbonate. The temperature of melamine precipitation depends from the cooling power of the cooling water, which is often taken from rivers like the Danube in Austria. This means that in winter it can be cooled to 20 °C whereby in summer cooling is often only down to 25°C.

In order to reduce the loss of guanidine carbonate and to keep the precipitation temperature of guanidine carbonate as low as possible (since precipitation of guanidine carbonate is also temperature dependent), currently a temperature difference of 3 to 5 °C between the melamine precipitation and the guanidine carbonate precipitation stage, in the sense that the temperature in the guanidine carbonate precipitation stage is higher than the temperature in the melamine precipitation stage, is applied. This is due to the fact that if the precipitation temperature of guanidine carbonate is below the precipitation temperature of melamine, then melamine and by-products like ammeline and ammelide would precipitate with guanidine carbonate what in turn leads to quality problems. The current specification shows that these by-products are nevertheless part of the guanidine carbonate product.

The object of the present invention is to provide a method which allows for increasing the guanidine carbonate yield and purity in an existing guanidine carbonate work-up or treatment process and to reduce the waste water of such a process.

This object is solved by a method according to claim 1.

Accordingly, a method for increasing the guanidine carbonate yield and purity from an aqueous solution containing melamine and guanidine carbonate in a guanidine carbonate treatment process is provided,

wherein the guanidine carbonate treatment process comprises at least one melamine precipitation stage and at least one guanidine carbonate precipitation stage; wherein ammonia is added to the guanidine carbonate precipitation stage;

wherein the temperature in the melamine precipitation stage is between 14°C and 30°C, and

wherein the temperature in the guanidine carbonate precipitation stage is at least 6°C, preferably at least 8°C, more preferably at least 10°C higher than in the melamine precipitation stage.

The present method allows for an increase of guanidine carbonate yield with improved product quality. Furthermore, the waste water output in the melamine low pressure plant can be reduced due to recycling of guanidine carbonate mother liquor and at the same time the product output of guanidine carbonate and melamine can be increased. In addition, the handling of the operations of the unit is improved since a higher temperature difference between the melamine precipitation stage and the guanidine carbonate precipitation stage of for example 10°C is easier to handle in a large scale compared to a lower temperature difference of 3-5°C. Therefore plant reliability is increased and off-spec production reduced.

The present method improves the guanidine carbonate yield in the melamine low pressure plant and surprisingly improves the quality (purity) of the guanidine carbonate at the same time. Furthermore, it is also possible to reduce the mother liquor disposal. This is surprisingly achieved by increasing the temperature difference between the melamine precipitation stage and the guanidine carbonate precipitation stage. Although at higher temperatures the solubility of the guanidine carbonate is increased and, relatively speaking, more guanidine carbonate remains in the mother liquor, the guanidine carbonate yield can still be increased, since the concentration of guanidine carbonate in the mother liquor is significantly higher due to a higher recycling rate. Thus, the precipitation temperature of guanidine carbonate becomes less important since less mother liquor containing guanidine carbonate is discharged with waste water and subsequently, a balance is reached. This leads to an overall increase of guanidine carbonate yield, since the guanidine carbonate precipitation (followed by centrifugation and drying) is the only way for the guanidine carbonate to leave the system.

Another beneficial effect is that the contribution of the melamine low pressure plant to the waste water can be reduced and in addition to an increase of guanidine carbonate yield also approx. 40 tons per year of melamine (and by-products) can be recycled and will not end up in the waste water.

In an embodiment of the present method the temperature difference between the at least one melamine precipitation stage and the at least one guanidine carbonate precipitation stage is in a range between 6 and 20°C, preferably between 8 and 15°C, more preferably between 10 and 12°. A temperature difference of about 10°C is in particular preferred in order to meet and improve the specification of the guanidine carbonate product.

As mentioned above, the temperature in the melamine precipitation stage is according to the invention between 14°C and 30°C, preferably between 17 and 27°C. A temperature of about 22°C is in particular preferred in the melamine precipitation stage.

In still a further embodiment of the present method the temperature in the guanidine carbonate precipitation stage is between 20°C and 50°C, preferably between 23°C and 40°C, more preferably between 26 and 35°C. A temperature of about 32°C is in particular preferred in the guanidine precipitation stage.

In a most preferred embodiment, a melamine precipitation temperature of 22°C and a precipitation temperature of 32°C in case of guanidine carbonate was selected, what is much higher than the original 3 to 5°C spread. In order to maintain the appropriate quality, it is necessary to strictly adhere to the required temperature parameters.

As mentioned previously the guanidine carbonate is obtained in a combined melamine low pressure process and guanidine carbonate treatment process. Thus, the aqueous solution containing melamine and guanidine carbonate comprises melamine mother liquor (MML) obtained in a melamine process and guanidine carbonate mother liquor (GCML) obtained in the guanidine carbonate treatment process.

In general a melamine low pressure process comprises the steps of:

- introducing urea and ammonia in a decomposer;
- introducing decomposed mixture containing isocyanic acid into the fixed-bed catalytic reactor (preferred catalyst $Al_2O_3$);
- introducing reaction mixture leaving the fixed bed catalytic reactor into a quencher;
- removal of melamine from the quenching mixture leaving the quencher by centrifugation, and
- providing melamine mother liquor (MML).

In the decomposer liquid urea is decomposed in a fluidized bed reactor (370 °C) to ammonia and isocyanic acid. Ammonia is used as the fluidizing gas. The gas stream is then fed to the fixed-bed catalytic reactor, where isocyanic acid is converted to melamine. Melamine is recovered from the reaction gas by quenching with water and mother liquor from the centrifuges.

The quencher is specially designed to work quickly, thereby preventing significant hydrolysis of melamine to ammelide and ammeline. In the quencher an aqueous melamine suspension is used to cool the reaction gases to 78 °C were solid melamine is obtained. A continuous circulation is carried out, which results in a 20% melamine suspension. The melamine suspension from the quencher is cooled further to 38 °C to complete the melamine crystallization process. The melamine is subsequently separated (for example by centrifugation) and no further melamine purification is necessary.

Thus, the melamine mother liquor (MML) containing guanidine carbonate is preferably obtained in a melamine low pressure process comprising the steps:

- introducing urea and ammonia into at least one decomposer for obtaining a decomposed mixture containing isocyanic acid;
- introducing the decomposed mixture containing isocyanic acid into at least one fixed-bed catalytic reactor wherein a melamine containing reaction mixture is formed;
- introducing the melamine containing reaction mixture into at least one quencher;
- removing melamine suspension (quenching mixture) from the quencher;
- separating melamine from the quenching mixture, preferably by centrifugation), and
- providing the melamine mother liquor (MML).

Guanidine carbonate is formed as a by-product of the melamine low pressure process. Further main by-products of the melamine low pressure process are ammeline, ammelide, cyanuric acid and cyano melamine.

Thus, the melamine mother liquor (MML) may comprise urea, melamine, ammonia, ammonium carbonate and guanidine carbonate, in particular said liquor may comprise 10 - 150 g/l, preferably 30 -70 g/l urea, 2 - 10 g/l, preferably 4 - 6 g/l melamine, 50 - 300 g/l, preferably 100 - 200 g/l, more preferably 140 -150 g/l ammonia and 5 - 60 g/l, preferably 15 - 35 g/l guanidine carbonate. For example, the melamine mother liquor (MML) may contain 20 g/l guanidine carbonate, 50 g/l urea, 5 g/l melamine and 140 g/l ammonia.

The guanidine carbonate formed in the course of the melamine process is dissolved in the melamine mother liquor. The mother liquor from the melamine filtration is partially used in the quenching process and the rest is worked up to obtain guanidine carbonate in the guanidine carbonate treatment process.

The melamine mother liquor is subsequently channeled into the guanidine carbonate treatment process. Thus, the guanidine carbonate treatment process is combined with a melamine process.

The guanidine carbonate treatment process comprises the steps of:

- combining melamine mother liquor (MML) and guanidine carbonate mother liquor (GCML) and exposing the combined mother liquors to an evaporation step in a mother liquor evaporation tank;
- cooling the concentrated combined mother liquors whereby melamine precipitates (melamine precipitation stage);
- removing precipitated melamine, preferably by filtration;
- adding ammonia to the mother liquor for precipitating guanidine carbonate (guanidine precipitation stage) and removing precipitated guanidine carbonate, preferably by centrifugation, and
- providing the guanidine carbonate mother liquor (GCML), which is at least in part recycled into the evaporation step.

As mentioned, at least a part of the guanidine carbonate mother liquor (GCML) is recycled within the guanidine carbonate treatment process (back into the evaporation stage). In an embodiment at least 60%, preferably at least 80%, more preferably to 100% of the guanidine carbonate mother liquor (GCML) are recycled within the guanidine carbonate treatment process. In a preferred embodiment none of the guanidine carbonate mother liquor (GCML) is disposed from the guanidine carbonate treatment process; i.e. 100% of GCML are recycled.

It is also possible that recycling phases and non-recycling phases iterate. For example, it is possible that guanidine carbonate mother liquor (GCML) is recycled with a recycling rate of 100% within the guanidine carbonate treatment process for a predetermined time period followed by a recycling rate of at least 60%, preferably at least 90% for a time period which is less than the time period for the recycling rate of 100%.

In another preferred embodiment only a small amount of guanidine carbonate mother liquor is continuously discarded. For example, between 2 and 10%, preferably between 3 and 5 % of guanidine carbonate mother liquor may be continuously discarded.

The guanidine carbonate mother liquor (GCML) comprises urea, melamine, ammonia and guanidine carbonate, preferably said liquor comprises 30 - 150 g/l, preferably 70 - 100 g/l urea, 2 - 8 g/l, preferably 3 - 5 g/l melamine, 100 - 350 g/l, preferably 180 - 270 g/l, more preferably 200 - 250 g/l ammonia and 10 - 60 g/l, preferably 20 - 40 g/l guanidine carbonate, for example 32 g/l guanidine carbonate.

The guanidine carbonate yield may be increased even further by adding cyanamide, dicyandiamide and/or ammonium carbonate (or alternatively ammonia / carbon dioxide) to

> a) the melamine mother liquor (MML) obtained in the melamine process,
> b) the guanidine carbonate mother liquor (GCML) obtained in the guanidine carbonate treatment process, or
> c) the mixture of a melamine mother liquor (MML) and a guanidine carbonate mother liquor (GCML).

A direct addition of cyanamide or dicyandiamide in combination with ammonium carbonate and ammonia (ammonium carbonate and ammonia are already present in the mother liquor, but can be added also additionally) form additional guanidine carbonate. By adding ammonium carbonate the formation of guanidine carbonate can be improved even further since the reaction equilibrium is influenced in favor of guanidine carbonate.

Since cyanamide or dicyandiamide are not completely converted to guanidine carbonate it has been shown to be an advantage that the guanidine carbonate mother liquor is recycled in the guanidine carbonate treatment process (as outlined above). Thus, the reduction of guanidine carbonate mother liquor disposal to as little as 10%, preferably to 3% or even zero provides an additional increase in the synthesis of guanidine carbonate since non-reacted cyanamide or dicyandiamide are kept within the cycle.

In an embodiment fresh melamine mother liquor may be added to the guanidine carbonate mother liquor before or after guanidine carbonate mother liquor is mixed with cyanamide and/or dicyandiamide and ammonium carbonate. In one variant the fresh melamine mother liquor may be added before or during the evaporation step of melamine mother liquor and guanidine carbonate mother liquor.

In an embodiment of the present method - after the addition of cyanamide and/or dicyandiamide and ammonium carbonate which can be also done either by adding ammonium carbonate or carbon dioxide and ammonia to the guanidine carbonate mother liquor (GCML) - the guanidine carbonate mother liquor (GCML) containing cyanamide and/or dicyandiamide and optionally ammonium carbonate is mixed with fresh melamine mother liquor (MML) and the mixture is reintroduced into the evaporation step.

In yet another embodiment of the present method the guanidine carbonate mother liquor (GCML) is mixed with fresh melamine mother liquor (MML), subsequently cyanamide and/or dicyandiamide and optionally ammonium carbonate are added and the mixture is reintroduced into the evaporation step.

In still another embodiment of the present method cyanamide and/or dicyandiamide and optionally ammonium carbonate are added to melamine mother liquor (MML), subsequently guanidine carbonate mother liquor (GCML) is added and the mixture is reintroduced into the evaporation step.

In case no additional ammonium carbonate is added the melamine mother liquor and the guanidine carbonate mother liquor are mixed before the addition of cyanamide and/or dicyandiamide.

It is also preferred, if the evaporation step of the melamine mother liquor (MML) from the melamine process (as part of the guanidine carbonate treatment process) in combination with the guanidine carbonate mother liquor (GCML) is carried out at 80°C - 150°C, preferably between 90°C - 125°C, more preferably 100°C - 120°C, at a pressure between 0.4 - 0.7 bar for a time of 1 - 6 h, preferably 2 - 4 h. The evaporation time depends on the evaporation temperature. For example, with an evaporation temperature of 130°C an evaporation time of 2 - 4h is sufficient. In case of an evaporation temperature of about 110°C the evaporation time may be between 5 - 6 h.

It has been shown that a low temperature and/or only a low overpressure have a positive impact on the overall yield. Thus, in a preferred embodiment the evaporation step may be carried out at 110 - 115°C and at a pressure of about 0.5 - 0.55bar.

After evaporation and removal of any precipitated melamine the remaining liquor is cooled down, preferably to 45°C or lower, as required in the guanidine carbonate precipitation stage. As pointed out above ammonia is added to the guanidine carbonate precipitation stage. Here in an embodiment of the present method 15 - 30 wt%, preferably 20 - 25wt%, based on the total amount of the mother liquor, gaseous or liquid ammonia are added to the mother liquor for precipitation of guanidine carbonate, wherein the addition of gaseous ammonia is preferred. The addition of ammonia increases the pH-value thereby reducing solubility of guanidine carbonate.

In a further embodiment of the present method 2.5 - 50 g/l cyanamide and/or 2.5 - 50 g/l dicyandiamide, preferably 5 - 25 g/l, more preferably 10 - 20 g /l cyanamide and/or 5 - 25 g/l, more preferably 10 - 20 g/l dicyandiamide are added.

In one embodiment cyanamide may be added in an amount between 0.05 - 1.2 mol/l, preferably 0.1 - 0.6 mol/l, more preferably between 0.2 - 0.5 mol/l.

In another embodiment dicyandiamide may be added in an amount between 0.03 - 0.6 mol/l, preferably between 0.05 - 0.3mol/l, preferably between 0.06 - 0.15 mol/l, for example dicyandiamide may be added in an amount between 0.1 - 0.3 mol/l.

The amount of ammonium carbonate that may be added to the respective mother liquors can be between 2.5 - 100 g/l, preferably 5 - 50 g/l, more preferably 10 - 30 g/l.

In an even more preferred embodiment of the present method 2.5 - 25 g/l cyanamide and/or 2.5 - 25 g/l dicyandiamide and 2.5 - 50 g/l ammonium carbonate are added to the melamine mother liquor (MML).

In another preferred embodiment of the present method 5 - 50 g/l cyanamide and/or 5 - 50 g/l dicyandiamide and 5-100 g/l ammonium carbonate are added to the guanidine carbonate mother liquor (GCML).

The specific advantage of the present process plus the addition of cyanamide and/or dicyanamide is, that due to the recycling there is no need that said substances require a 100% conversion rate to guanidine carbonate due to the recycling.

In a further variant of the present method the addition of cyanamide and/or dicyandiamide and optionally ammonium carbonate to the melamine mother liquor (MML), a guanidine carbonate mother liquor (GCML) or a mixture thereof is carried out at a temperature between 100°C and 150°C, preferably between 110°C and 140°C, for 15 to 120 min. Said temperature range is also applied in all subsequent reaction steps, in particular in the guanidine carbonate treatment process.

The above described method may be carried out in existing melamine and guanidine carbonate treatment plants. In one embodiment cyanamide, dicyandiamide and optionally ammonium carbonate may be introduced into a tank containing melamine mother liquor (MML), into a tank containing a guanidine carbonate mother liquor (GCML), and/or into an evaporation reactor containing a mixture of both melamine mother liquor (MML) and a guanidine carbonate mother liquor (GCML). The addition of cyanamide, dicyandiamide and optionally ammonium carbonate to a MML tank and/or a GCML tank are preferred.

However, it is also conceivable and possible to introduce cyanamide, dicyandiamide and optionally ammonium carbonate (and also fresh melamine mother liquor) into a separate reactor, such a tubular reactor or continuous stirred tank reactor, which is located between the tank containing a guanidine carbonate mother liquor (GCML) and the evaporation tank, i.e. downstream of the GCML tank and upstream of the evaporation reactor.

In summary, there are at least four different locations within the plant system, at which cyanamide, dicyandiamide and optionally ammonium carbonate may be added to the melamine mother liquor (MML), guanidine carbonate mother liquor or a mixture of both. In a first embodiment cyanamide, dicyandiamide and optionally ammonium carbonate are introduced into the melamine mother liquor tank. In a second embodiment cyanamide, dicyandiamide and optionally ammonium carbonate are introduced into the guanidine carbonate mother liquor tank. In a third embodiment cyanamide, dicyandiamide and optionally ammonium carbonate are introduced into the evaporation reactor. And in a fourth embodiment cyanamide, dicyandiamide and optionally ammonium carbonate are introduced into the tubular line (or tubular reactor) or batch reactor upstream or upfront of the guanidine carbonate mother liquor evaporation reactor.

As stated previously, guanidine carbonate mother liquor may be mixed with fresh melamine mother liquor before or after adding cyanamide, dicyandiamide and optionally ammonium carbonate. Thus, in one embodiment the fresh melamine mother liquor may be introduced into the evaporation reactor. In a second embodiment the fresh melamine mother liquor may be introduced into the tubular line (or tubular reactor) or batch reactor between guanidine carbonate mother liquor tank and mother liquor evaporation reactor.

The present method allows for providing guanidine carbonate of high purity that is purity higher than 99.7 %, preferably higher than 99.8%, in particular preferably higher than 99.85%. The purity of guanidine carbonate was determined by HPLC as described in the experimental section.

The invention is explained in more detail by means of the following examples with reference to the Figures. It shows:

Figure 1     a scheme of a combined melamine low pressure process and a guanidine carbonate treatment process; and

Figure 2     a diagram illustrating the yield of guanidine carbonate as obtained in a test run as described in the example.

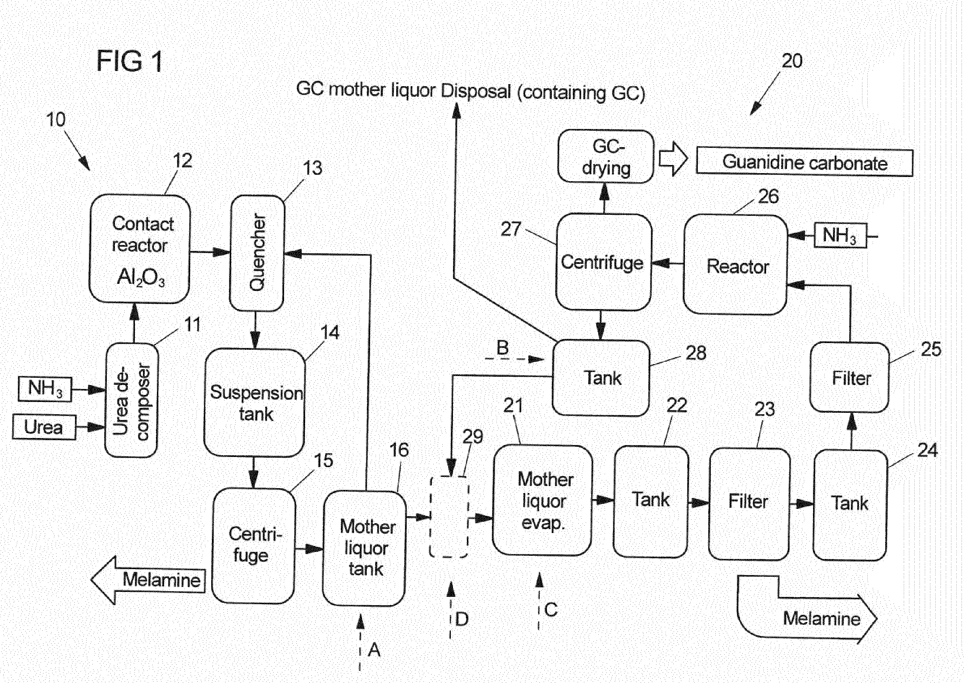

Figure 1 shows a melamine low pressure process 10 that is connected to a process for isolating guanidine carbonate 20 (guanidine carbonate treatment process).

The central steps of a melamine low pressure process are: introducing urea and ammonia in a decomposer 11; introducing decomposed mixture containing isocyanic acid into the fixed-bed catalytic reactor 12; introducing reaction mixture leaving the fixed bed catalytic reactor 12 into a quencher 13; removal of melamine from the quenching mixture leaving the quencher 13 by centrifugation 15; and providing melamine mother liquor (MML) in a mother liquor tank 16.

Urea and ammonia are introduced in the decomposer 11 and decomposed in a fluidised bed reactor (370 °C) to ammonia and isocyanic acid. Ammonia is used as the fluidizing gas. Heat required for the decomposition is supplied to the reactor by hot molten salt circulated through internal heating coils.

The gas stream leaving the decomposer 11 is then fed to the fixed-bed catalytic reactor 12, where isocyanic acid is converted to melamine at ca. 450 °C and near-atmospheric pressure in the presence of $Al_2O_3$ as catalyst.

Melamine is recovered from the reaction gas by quenching with water and mother liquor in quencher 13. The quencher 13 is designed to work quickly, thereby preventing significant hydrolysis of melamine to ammelide and ammeline. In the quencher 13 an aqueous melamine suspension is used to cool the reaction gases to 78 °C whereby solid melamine is obtained. A continuous circulation is carried out, which results in a 20% melamine suspension.

The melamine suspension from the quencher 13 is cooled further to 38 °C in the suspension tank 14 to complete the melamine crystallization process. Subsequently, melamine is separated from the suspension in centrifuge 15.

Any guanidine carbonate formed in the course of the melamine process 10 (as described previously above) is dissolved in the melamine mother liquor. The mother liquor from the melamine filtration is partially used in the quenching process and the rest is worked up to obtain guanidine carbonate in the guanidine carbonate treatment process 20.

Here, the melamine mother liquor (MML) is transferred from the MML tank 16 to the evaporation tank 21 where the MML mixes with the recycled guanidine carbonate mother liquor (GCML). The combined mother liquors (MML and GCML) are heated in the evaporation reactor 21 to remove the rest of ammonia and carbon dioxide and are simultaneously concentrated, for example up to 20 % volume.

The concentrated combined MML + GCML are transferred to a further tank 22 for cooling off whereby most of the melamine contained therein precipitates. The precipitated melamine is filtered off using filter 23. After filtration the mother liquor goes to another tank 24 and is filtered again with filter 25. In reactor 26, gaseous ammonia is introduced whereby guanidine carbonate precipitates which can be separated in a centrifuge 27. The separated guanidine carbonate is channeled out of the process at this point.

Ammonia is added to precipitate the guanidine carbonate caused by an increase of the pH-value. The higher the ammonia content is the better the precipitation of guanidine carbonate works. The addition of ammonia leads to a temperature increase of the solution due to the high heat caused by ammonia absorption. Therefore another cooling step is necessary to cool the solution down. However, the precipitation temperature in the guanidine carbonate precipitation reactor 26 is above the temperature in the melamine precipitation in tank 22 otherwise not precipitated melamine or other impurities would be precipitated together with the guanidine carbonate.

According to an embodiment of the present method the temperature in the melamine precipitation in tank 22 is about 22°C, whereas the temperature in the guanidine carbonate precipitation reactor 26 is about 32°C.

The guanidine carbonate mother liquor leaving the centrifuge 27 is transferred to another tank 28. From tank 28 guanidine carbonate mother liquor is partly disposed as waste water.

The average mass balance of the composition of the disposed mother liquor in a state-of-the-art GC treatment process is illustrated in the following table 1:

Table 1

| Sample taken | Kg/h (average) |
|---|---|
| GC absolute | 45 |
| Urea absolute | 121 |
| Melamine | 5.99 |
| Ammeline | 0.36 |
| Ammelide | 0.3 |
| Cyanic acid | 0.14 |
| Cyan melamine | 0.85 |
| Ureido melamine | - |
| Melem | 0.01 |
| Melam | 0.01 |

This amounts to a yearly disposal of guanidine carbonate mother liquor with the following amounts (24 h/day for 300 days):

Table 2

| GC [t/year] | Melamine [t/year] | Ammeline [t/year] | Ammelide [t/year] | Cyanuric acid [t/year] | Cyano melamine [t/year] |
|---|---|---|---|---|---|
| 324 | 43 | 2,6 | 2,2 | 1,0 | 6,1 |

The other (non-disposed) part guanidine carbonate mother liquor is passed to an additional reactor or tube 29 and is subsequently reintroduced into the mother liquor evaporation reactor 21.

According to an embodiment of the present method the disposal of guanidine carbonate mother liquor from tank 28 is drastically reduced; i.e. only a small amount of GCML is disposed or none at all. In the latter case all guanidine carbonate mother liquor from tank 28 is recycled back to the evaporation tank 21.

In this manner not only the guanidine carbonate yield can be increased, but also the amount of waste water can be reduced by 1200 l/h and approx. 40 tons/year of melamine (and by-products) can be recycled and will not end up in the waste water.

Cyanamide, dicyandiamide and optionally ammonium carbonate may be added at different locations (depicted as dashed arrows A-D in Figure 1).

In a first setting A cyanamide, dicyandiamide and optionally ammonium carbonate are introduced into the melamine mother liquor tank 16. In a second setting B cyanamide, dicyandiamide and optionally ammonium carbonate are introduced into the guanidine carbonate mother liquor tank 28. In a third setting C cyanamide, dicyandiamide and optionally ammonium carbonate are introduced into the mother liquor evaporation reactor 21. And in a fourth setting D cyanamide, dicyandiamide and optionally ammonium carbonate are introduced into the tubular line, tubular or batch reactor 29 installed between tanks 28 and 21.

## Example

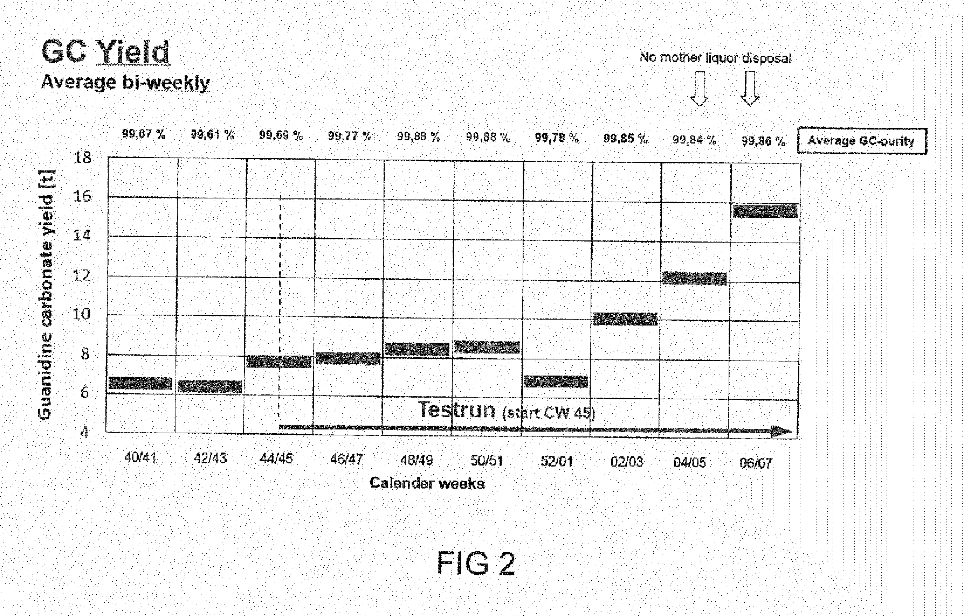

The data provided in Table 3 below and illustrated in diagram of Figure 2 were obtained conducting test runs with varying temperatures in the guanidine carbonate precipitation reactor 26 and varying amounts of guanidine carbonate mother liquor disposed from tank 28.

In a conventional run (calendar weeks 40-44) the temperature in the guanidine carbonate precipitation reactor 26 was maintained at 30°C and 1200 l/h of guanidine carbonate mother liquor were disposed as waste water.

When starting the test run the temperature in the guanidine carbonate precipitation reactor 26 was increased to 35°C (calendar weeks 45-5) and reduced to 33°C (calendar weeks 6-7). Simultaneously the disposal of guanidine carbonate mother liquor was gradually reduced from 1200 l/h to 0 l/h.

As a result the stepwise reduction (1200 → 750 → 500 → 250 → 0 l/h) of mother liquor disposal and the increase of recycle to the evaporation stage showed an increase in the guanidine carbonate yield. Moreover the GC quality was surprisingly improved although the mother liquor disposal was reduced to zero.

This was possible because a minimum temperature difference of 10 °C was maintained between precipitation of melamine (and other by-products) and the precipitation of GC.

Table 3

| | C W | Precip. Tin reactor 26 | Precip. Tin Tank 22 | GCML disposal | GC | GC bi-weekly average | GC quality bi-weekly average |
|---|---|---|---|---|---|---|---|
| | | °C | °C | l/h | kg | t | % |
| before Testrun (conventional run) | 40 | 30 | 25 | 1200 | 7350 | 6,525 | 99,67 |
| | 41 | 30 | 25 | 1200 | 5700 | | |
| | 42 | 30 | 25 | 1200 | 8250 | 6,375 | 99,61 |
| | 43 | 30 | 25 | 1200 | 4500 | | |
| | 44 | 30 | 25 | 1200 | 4500 | 7,725 | 99,69 |
| Testrun | 45 | 35 | 25 | 750 | 10950 | | |
| | 46 | 35 | 25 | 500 | 5250 | 7,875 | 99,77 |
| | 47 | 35 | 25 | 250 | 10500 | | |
| | 48 | 35 | 25 | 250/0 | 6000 | 8,4 | 99,88 |
| | 49 | 35 | 24 | 300 | 10800 | | |
| | 50 | 35 | 24 | 300-500 | 10800 | 8,55 | 99,88 |
| | 51 | 35 | 24 | 300-500 | 6300 | | |
| | 52 | 35 | 24 | 300-500 | 7350 | 6,825 | 99,78 |

(continued)

| | C W | Precip. Tin reactor 26 | Precip. Tin Tank 22 | GCML disposal | GC | GC bi-weekly average | GC quality bi-weekly average |
|---|---|---|---|---|---|---|---|
| | | °C | °C | l/h | kg | t | % |
| | 1 | 35 | 24 | 300-500 | 6300 | | |
| | 2 | 35 | 23 | 300-500 | 10500 | 10,05 | 99,85 |
| | 3 | 35 | 23 | 0 | 9600 | | |
| | 4 | 35 | 23 | 0 | 12600 | 12,15 | 99,83 |
| | 5 | 35 | 23 | 0-400 | 11700 | | |
| | 6 | 33 | 22 | 0 | 16200 | 15,6 | 99,86 |
| | 7 | 33 | 22 | 0-300 | 15000 | | |

**HPLC method for analysing purity of guanidine carbonate obtained in the process**

**Principle:**

Separation of by-products of GC by use of HPLC and evaluation of the chromatograms by external calibration method.

**Reagents:**

Water HPLC grade (MilliQ-Water); Primary potassium phosphate; $KH_2PO_4$ (Analytical grade), Sodium hydroxide, NaOH (Analytical grade); Hydrochloric acid HCl 6 mol/l

**Apparatus:**

Analytical balance Mettler AT 250; Ultra sonic bath; pH-meter with a combined glass pH electrode; Volumetric flasks 500 ml, 1000 ml; Pipettes; Vials for HPLC-autosampler with wide neck and septa of red rubber

HPLC-filter: Pore size 0.20 $\mu$m    e. g. Sartorius Minisart RC 15
Order number 17761_Q

**HPLC system,** consisting of:

Quaternary HPLC pump with degassing system (G 1354 A Agilent LC 1100)
Diode-Array detector (G 1315 - 64001 Agilent)
Automatic sampling system (G1313 A Agilent)
HPLC ChemStation Software
Column: ZORBAX Eclipse XDB- C 18, 250 x 4,6 mm , 5 $\mu$m
Eluent: Buffer solution at pH 7.5

500 ml of 0.1 molar $KH_2PO_4$ + 409 ml 0.1 molar NaOH
adjust to almost pH = 7.495 under stirring.

0.1 molar $KH_2PO_4$: Dissolve 13.609 g in 1000 ml water
0.1 molar NaOH: Dissolve 4.0 g NaOH in 1000 ml water

**Reference standards:**

Ammelide; Cyanuric acid; Ammeline; Melamine

**Reference standard solution:**

Weigh 2.0 mg each of Ammelide, Cyanuric acid, Ammeline and Melamine, put them into a 1000 ml volumetric flask, add about 980 ml of water (HPLC grade) and dissolve by warming up at about 50 - 60 °C and by the use of an ultra sonic bath. When completely dissolved cool the solution to 20 °C and fill up to 1000 ml with water (HPLC grade).
This solution of mixed reference standards is stored at room temperature and labelled as **"MSTD-GC".**

**Table of reference standards:**

| Reference standard | Reference standard solution Concentration mg / l (40 µl injected) | Retention time minutes | mg / 1000 g sample (ppm) calculated for sample weight of 1.0 g / 500 ml |
|---|---|---|---|
| Ammelide | 2.0 | 2.87 | **1000** |
| Cyanuric acid | 2.0 | 3.05 | **1000** |
| Ammeline | 2.0 | 3.4 | **1000** |
| Melamine | 2.0 | 4.86 | **1000** |

**Calculation:**

$$\text{Content of by-product in mg/ 1000g (ppm)} = \frac{A \times 1000}{B}$$

A    Reference standard in mg / l
B    Sample weight in g / l

**Procedure:**

Weigh 1.0 g of the homogenous sample into a 500 ml volumetric flask, add approximately 300 ml water (HPLC grade) and 1 ml 6 mol/l HCl and fill up to 500 ml with water (HPLC grade). Dissolve for one minute in the ultrasonic bath. When completely dissolved filter 2 ml of the solution by the HPLC-filter device (20 µm pore size) into an autosampler vial. Close the vial and inject 40 µl of the content according to the HPLC-method "GC_NEW.M".

**HPLC-Methode "GC NEW":**

| | |
|---|---|
| Column: | ZORBAX Eclipse XDB- C 18, 250 x 4,6 mm , 5 µm |
| Temperature of the column: | 20°C |
| Injection volume: | 40 µl |
| Mobile phase: | Buffer solution pH = 7.5 |
| Flow rate: | 1.0 ml / min |
| Detector: | Diode array at 220 nm wavelength |

As can be seen from table 3 above both the GC yield (GC bi-weekly average) and the GC purity (quality bi-weekly average) were increased with increasing temperature difference between the guanidine carbonate precipitation and the melamine precipitation according to the claimed invention.

**Claims**

1. Method for increasing the guanidine carbonate yield and purity from an aqueous solution containing melamine and guanidine carbonate in a guanidine carbonate treatment process (20),

wherein the guanidine carbonate treatment process (20) comprises at least one melamine precipitation stage (22) and at least one guanidine carbonate precipitation stage (26), wherein ammonia is added to the guanidine carbonate precipitation stage (26);

wherein the temperature in the melamine precipitation stage (22) is between 14°C and 30°C, and

wherein the temperature in the guanidine carbonate precipitation stage (26) is at least 6°C higher than in the melamine precipitation stage (22).

2. Method according to claim 1, **characterized in that** the temperature difference between the at least one melamine precipitation stage (22) and the at least one guanidine carbonate precipitation stage (26) is in a range between 6 and 20°C.

3. Method according to one of the preceding claims, **characterized in that** the temperature in the melamine precipitation stage (22) is between 17 and 27°C.

4. Method according to one of the preceding claims, **characterized in that** the temperature in the guanidine carbonate precipitation stage (26) is between 20°C and 50°C.

5. Method according to one of the preceding claims, **characterized in that** the aqueous solution containing melamine and guanidine carbonate comprises melamine mother liquor (MML) obtained in a melamine process (10) and guanidine carbonate mother liquor (GCML) obtained in the guanidine carbonate treatment process (20).

6. Method according to claim 5, **characterized in that** at least 60% of the guanidine carbonate mother liquor (GCML) are recycled within the guanidine carbonate treatment process (20).

7. Method according to claim 6, **characterized in that** none of the guanidine carbonate mother liquor (GCML) is disposed from the guanidine carbonate treatment process (20).

8. Method according to claim 6, **characterized in that** the guanidine carbonate mother liquor (GCML) is recycled with a recycling rate of 100% within the guanidine carbonate treatment process (20) for a predetermined time period followed by a recycling rate of at least 60% for a time period which is less than the time period for the recycling rate of 100%.

9. Method according to one of the preceding claims, **characterized in that** guanidine carbonate mother liquor (GCML) is obtained in the guanidine carbonate treatment process (20) comprising the steps of:

- combining melamine mother liquor (MML) and guanidine carbonate mother liquor (GCML) and exposing the combined mother liquors to an evaporation step in a mother liquor evaporation tank (21);
- cooling the concentrated combined mother liquors whereby melamine precipitates (melamine precipitation stage 22);
- removing precipitated melamine;
- adding ammonia to the mother liquor for precipitating guanidine carbonate (guanidine precipitation stage 26) and removing precipitated guanidine carbonate, preferably by centrifugation, and
- providing the guanidine carbonate mother liquor (GCML), which is at least partly recycled into the evaporation step.

10. Method according to claim 9, **characterized in that** 15-30 wt% based on the total amount of the mother liquor, gaseous or liquid ammonia are added to the mother liquor for precipitation of guanidine carbonate.

11. Method according to one of claims 5 to 10, **characterized in that** the guanidine carbonate mother liquor (GCML) comprises urea, melamine, ammonia and guanidine carbonate.

12. Method according to one of the preceding claims, **characterized in that** the guanidine carbonate treatment process (20) is combined with a melamine process (10) such that the melamine mother liquor (MML) obtained in the melamine process is channeled into the guanidine carbonate treatment process (20).

13. Method according to one of claims 5 to 12, **characterized in that** the melamine mother liquor (MML) is obtained in a melamine low pressure process comprising the steps:

- introducing urea and ammonia into at least one decomposer for obtaining a decomposed mixture containing isocyanic acid;
- introducing the decomposed mixture containing isocyanic acid into at least one fixed-bed catalytic reactor wherein a melamine containing reaction mixture is formed;
- introducing the melamine containing reaction mixture into at least one quencher;
- removing melamine suspension from the quencher, and
- providing the melamine mother liquor (MML).

**14.** Method according to one of claims 5 to 12, **characterized in that** the melamine mother liquor (MML) comprises urea, melamine, ammonia and guanidine carbonate.

**Patentansprüche**

**1.** Verfahren zum Erhöhen der Ausbeute und Reinheit von Guanidincarbonat aus einer wässrigen Lösung, die Melamin und Guanidincarbonat enthält, in einem Guanidincarbonat-Behandlungsverfahren (20),

wobei das Guanidincarbonat-Behandlungsverfahren (20) mindestens eine Melaminausfällungsstufe (22) und mindestens eine Guanidincarbonat-Ausfällungsstufe (26) umfasst, wobei der Guanidincarbonat-Ausfällungsstufe (26) Ammoniak zugesetzt wird;
wobei die Temperatur in der Melaminausfällungsstufe (22) zwischen 14°C und 30°C beträgt, und
wobei die Temperatur in der Guanidincarbonat-Ausfällungsstufe (26) mindestens 6°C höher ist als in der Melaminausfällungsstufe (22).

**2.** Verfahren nach Anspruch 1, **dadurch gekennzeichnet, dass** der Temperaturunterschied zwischen der mindestens einen Melaminausfällungsstufe (22) und der mindestens einen Guanidincarbonat-Ausfällungsstufe (26) in einem Bereich zwischen 6 und 20°C liegt.

**3.** Verfahren nach einem der vorhergehenden Ansprüche, **dadurch gekennzeichnet, dass** die Temperatur in der Melaminausfällungsstufe (22) zwischen 17 und 27°C beträgt.

**4.** Verfahren nach einem der vorhergehenden Ansprüche, **dadurch gekennzeichnet, dass** die Temperatur in der Guanidincarbonat-Ausfällungsstufe (26) zwischen 20°C und 50°C beträgt.

**5.** Verfahren nach einem der vorhergehenden Ansprüche, **dadurch gekennzeichnet, dass** die Melamin und Guanidincarbonat enthaltende wässrige Lösung in einem Melaminverfahren (10) erhaltene Melaminmutterlauge (MML) und in dem Guanidincarbonat-Behandlungsverfahren (20) erhaltene Guanidincarbonatmutterlauge (GCML) umfasst.

**6.** Verfahren nach Anspruch 5, **dadurch gekennzeichnet, dass** mindestens 60% der Guanidincarbonatmutterlauge (GCML) innerhalb des Guanidincarbonat-Behandlungsverfahrens (20) wiederverwertet werden.

**7.** Verfahren nach Anspruch 6, **dadurch gekennzeichnet, dass** nichts von der Guanidincarbonatmutterlauge (GCML) bei dem Guanidincarbonat-Behandlungsverfahren (20) verwertet wird.

**8.** Verfahren nach Anspruch 6, **dadurch gekennzeichnet, dass** die Guanidincarbonatmutterlauge (GCML) mit einer Recyclingquote von 100% im Rahmen des Guanidincarbonat-Behandlungsverfahrens (20) über eine vorbestimmte Zeit wiederverwertet wird, gefolgt von einer Recyclingquote von mindestens 60% über eine Zeit, die kleiner ist als die Zeit für die Recyclingquote von 100%.

**9.** Verfahren nach einem der vorhergehenden Ansprüche, **dadurch gekennzeichnet, dass** man Guanidincarbonatmutterlauge (GCML) bei dem Guanidincarbonat-Behandlungsverfahren (20) erhält, das die folgenden Schritte umfasst:

- Vereinigen von Melaminmutterlauge (MML) und Guanidincarbonatmutterlauge (GCML) und Unterziehen der vereinigten Mutterlaugen einem Verdampfungsschritt in einem Mutterlaugenverdampfungsbehälter (21);
- Abkühlen der konzentrierten vereinigten Mutterlaugen, wodurch Melamin ausfällt (Melaminausfällungsstufe 22);

- Entfernen des ausgefällten Melamins;
- Zugabe von Ammoniak zu der Mutterlauge, um Guanidincarbonat auszufällen (Guanidinausfällungsstufe 26), und Entfernen des ausgefällten Guanidincarbonats, vorzugsweise durch Zentrifugieren, und
- Bereitstellen der Guanidincarbonatmutterlauge (GCML), die wenigstens teilweise in den Verdampfungsschritt zurückgeführt wird.

10. Verfahren nach Anspruch 9, **dadurch gekennzeichnet, dass** 15-30 Gew.-%, bezogen auf die Gesamtmenge an Mutterlauge, von gasförmigem oder flüssigem Ammoniak der Mutterlauge zugesetzt werden, um Guanidincarbonat auszufällen.

11. Verfahren nach einem der Ansprüche 5 bis 10, **dadurch gekennzeichnet, dass** die Guanidincarbonatmutterlauge (GCML) Harnstoff, Melamin, Ammoniak und Guanidincarbonat umfasst.

12. Verfahrennach einem der vorhergehenden Ansprüche, **dadurch gekennzeichnet, dass** das Guanidincarbonat-Behandlungsverfahren (20) mit einem Melaminverfahren (10) kombiniert wird, sodass die bei dem Melaminverfahren erhaltene Melaminmutterlauge (MML) in das Guanidincarbonat-Behandlungsverfahren (20) eingeleitet wird.

13. Verfahren nach einem der Ansprüche 5 bis 12, **dadurch gekennzeichnet, dass** man die Melaminmutterlauge (MML) in einem Niederdruckmelaminverfahren erhält, das die folgenden Schritte umfasst:

- Einleiten von Harnstoff und Ammoniak in mindestens einen Zersetzer, um eine Isocyansäure enthaltende zersetzte Mischung zu erhalten;
- Einleiten der Isocyansäure enthaltenden zersetzten Mischung in mindestens einen katalytischen Festbettreaktor, wobei ein Melamin enthaltendes Reaktionsgemisch gebildet wird;
- Einleiten des Melamin enthaltenden Reaktionsgemisches in mindestens einen Quencher;
- Entfernen von Melaminsuspension aus dem Quencher, und
- Bereitstellen der Melaminmutterlauge (MML).

14. Verfahren nach einem der Ansprüche 5 bis 12, **dadurch gekennzeichnet, dass** die Melaminmutterlauge (MML) Harnstoff, Melamin, Ammoniak und Guanidincarbonat umfasst.

**Revendications**

1. Procédé permettant d'augmenter le rendement et la pureté du carbonate de guanidine à partir d'une solution aqueuse contenant de la mélamine et du carbonate de guanidine dans un procédé de traitement du carbonate de guanidine (20),

dans lequel le procédé de traitement du carbonate de guanidine (20) comprend au moins une étape de précipitation de mélamine (22) et au moins une étape de précipitation de carbonate de guanidine (26), de l'ammoniac étant ajouté à l'étape de précipitation de carbonate de guanidine (26) ;
dans lequel la température de l'étape de précipitation de mélamine (22) est comprise entre 14°C et 30°C, et dans lequel la température de l'étape de précipitation de carbonate de guanidine (26) est supérieure d'au moins 6°C à celle de l'étape de précipitation de mélamine (22).

2. Procédé selon la revendication 1, **caractérisé en ce que** la différence de température entre l'au moins une étape de précipitation de mélamine (22) et l'au moins une étape de précipitation de carbonate de guanidine (26) est comprise entre 6 et 20°C.

3. Procédé selon l'une quelconque des revendications précédentes, **caractérisé en ce que** la température dans l'étape de précipitation de mélamine (22) est comprise entre 17 et 27°C.

4. Procédé selon l'une quelconque des revendications précédentes, **caractérisé en ce que** la température de l'étape de précipitation de carbonate de guanidine (26) est comprise entre 20°C et 50°C.

5. Procédé selon l'une quelconque des revendications précédentes, **caractérisé en ce que** la solution aqueuse contenant de la mélamine et du carbonate de guanidine comprend de la liqueur mère de mélamine (LMM) obtenue dans un procédé de mélamine (10) et de la liqueur mère de carbonate de guanidine (LMCG) obtenue dans le procédé de traitement du carbonate de guanidine (20).

6. Procédé selon la revendication 5, **caractérise en ce qu'**au moins 60 % de la liqueur mère de carbonate de guanidine (LMCG) sont recyclés dans le procédé de traitement du carbonate de guanidine (20).

7. Procédé selon la revendication 6, **caractérisé en ce qu'**aucune liqueur mère de carbonate de guanidine (LMCG) n'est éliminée du procédé de traitement du carbonate de guanidine (20).

8. Procédé selon la revendication 6, **caractérisé en ce que** la liqueur mère de carbonate de guanidine (LMCG) est recyclée à un taux de recyclage de 100 % dans le procédé de traitement du carbonate de guanidine (20) pendant une période prédéterminée, suivie d'un taux de recyclage d'au moins 60 % pendant une période inférieure à celle du taux de recyclage de 100 %.

9. Procédé selon l'une quelconque des revendications précédentes, **caractérisé en ce que** la liqueur mère de carbonate de guanidine (LMCG) est obtenue dans le procédé de traitement du carbonate de guanidine (20) comprenant les étapes suivantes :

   - combiner la liqueur mère de mélamine (LMM) et la liqueur mère de carbonate de guanidine (LMCG) et exposer les liqueurs mères combinées à une étape d'évaporation dans un réservoir d'évaporation de la liqueur mère (21) ;
   - refroidir les liqueurs mères concentrées et combinées par précipitation de mélamine (étape 22 de la précipitation de mélamine) ;
   - éliminer la mélamine précipitée ;
   - ajouter de l'ammoniac à la liqueur mère pour précipiter du carbonate de guanidine (étape 26 de la précipitation de guanidine) et éliminer du carbonate de guanidine précipité, de préférence par centrifugation, et
   - fournir la liqueur mère de carbonate de guanidine (LMCG), qui est au moins partiellement recyclée dans l'étape d'évaporation.

10. Procédé selon la revendication 9, **caractérisé en ce que** 15 à 30 % en poids de la quantité totale de liqueur mère, de l'ammoniac gazeux ou liquide sont ajoutés à la liqueur mère pour la précipitation de carbonate de guanidine.

11. Procédé selon l'une quelconque des revendications 5 à 10, **caractérisé en ce que** la liqueur mère de carbonate de guanidine (LMCG) comprend de l'urée, de la mélamine, de l'ammoniac et du carbonate de guanidine.

12. Procédé selon l'une quelconque des revendications précédentes, **caractérisé en ce que** le procédé de traitement du carbonate de guanidine (20) est combiné avec un procédé de mélamine (10) de sorte que la liqueur mère de mélamine (LMM) obtenue dans le procédé de mélamine est canalisée dans le procédé de traitement du carbonate de guanidine (20).

13. Procédé selon l'une quelconque des revendications précédentes, **caractérisé en ce que** la liqueur mère de mélamine (LMM) est obtenue dans un procédé de mélamine à basse pression comprenant les étapes suivantes :

   - introduire de l'urée et de l'ammoniac dans au moins un décomposeur pour obtenir un mélange décomposé contenant de l'acide isocyanique ;
   - introduire le mélange décomposé contenant de l'acide isocyanique dans au moins un réacteur catalytique à lit fixe dans lequel se forme un mélange réactionnel contenant de la mélamine ;
   - introduire le mélange réactionnel contenant de la mélamine dans au moins un quencher;
   - éliminer la suspension de mélamine du quencher, et
   - fournir la liqueur mère de mélamine (LMM).

14. Procédé selon l'une quelconque des revendications 5 à 12, **caractérisé en ce que** la liqueur mère de mélamine (LMM) comprend de l'urée, de la mélamine, de l'ammoniac et du carbonate de guanidine.

**FIG 1**

10

**11** Urea de-composer ← NH₃, Urea

**12** Contact reactor Al₂O₃

**13** Quencher

**14** Suspension tank

**15** Centri-fuge → Melamine

**16** Mother liquor tank ← A

**29**

**21** Mother liquor evap. ← C, ← D

**22** Tank

**23** Filter → Melamine

**24** Tank

**25** Filter ← NH₃

**26** Reactor → Guanidine carbonate

**27** Centrifuge → GC-drying

**28** Tank

B

GC mother liquor Disposal (containing GC)

20

FIG 2

**REFERENCES CITED IN THE DESCRIPTION**

**Patent documents cited in the description**

- DE 938843 C
- US 2221478 A
- DE 1593472 A1
- AT 317918 B
- AT 336035 B
- US 3952057 A